# EUROPEAN PATENT SPECIFICATION

(11) **EP 1 506 998 B1**
(45) Date of publication and mention of the grant of the patent: **19.03.2008**
(21) Application number: 04103880.3
(22) Date of filing: 12.08.2004
(51) Int. Cl.: C12N 5/06, G01N 33/50

(54) **An in vitro tumor angiogenesis model**
Ein in vitro Modell der Tumorangiogenese
Un modèle in vitro d'angiogenèse tumorale

(30) Priority: 14.08.2003 US 495429 P; 06.08.2004 US 912904
(43) Date of publication of application: 16.02.2005
(73) Proprietor: Becton, Dickinson and Company, Franklin Lakes, New Jersey 07417-1880 (US)
(72) Inventor: Wu, Min, Carlisle, MA 01741 (US)
(74) Representative: Helbing, Jörg

(56) References cited:
- LAFLEUR MARC A ET AL: "Endothelial tubulogenesis within fibrin gels specifically requires the activity of membrane-type-matrix metalloproteinases (MT-MMPs)" JOURNAL OF CELL SCIENCE, vol. 115, no. 17, 1 September 2002 (2002-09-01), pages 3427-3438, XP002309413 ISSN: 0021-9533
- ABE T ET AL: "Induction of vascular endothelial tubular morphogenesis by human glioma cells. A model system for tumor angiogenesis" JOURNAL OF CLINICAL INVESTIGATION 1993 UNITED STATES, vol. 92, no. 1, 1993, pages 54-61, XP002309414 ISSN: 0021-9738
- NAKAGAWA M ET AL: "Tubulogenesis by microvascular endothelial cells is mediated by vascular endothelial growth factor (VEGF) in renal cell carcinoma." BRITISH JOURNAL OF UROLOGY. MAY 1997, vol. 79, no. 5, May 1997 (1997-05), pages 681-687, XP002309415 ISSN: 0007-1331

## Description

### BACKGROUND OF THE INVENTION

### Field of the Invention

This application claims priority of U.S. Provisional Patent Application No. 60/495,429, filed August 14, 2003.

The present invention relates to a method of inducing tubulogenesis in normal endothelial cells.

### Background of the Invention

Endothelial cells that form the lining of blood vessels are well known for their capacity to adjust their numbers and arrangement to suite local requirements. All tissues depend on a blood supply and the blood supply depends on endothelial cells. Blood vessels create an adaptable life support system in every region of the body. If not for endothelial cells extending and maintaining this network of blood vessels, tissue growth and repair would not be possible.

The largest blood vessels are arteries and veins, which have a thick tough outer wall of connective tissue and smooth muscle. The wall is lined by a thin single layer of endothelial cells, separated from the surrounding outer layers by a basal lamina. While the amounts of connective-tissue and smooth muscle in the vessel wall may vary according to the vessel's diameter and function, the endothelial lining is always present. In the smaller capillaries and sinusoids, the walls consist solely of endothelial cells and basal lamina. Thus, endothelial cells line the entire vascular system. Studies have shown that arteries and veins develop from small vessels constructed solely of endothelial cells and a basal lamina, connective tissue and smooth muscle being added later where required upon signals from the endothelial cells.

Throughout the vascular system endothelial cells retain a capacity for cell division and movement. This is important in repair and maintenance of the vascular system. For example, if a part of the wall of a blood vessel is damaged and loses endothelial cells, neighboring endothelial cells will proliferate and migrate in to cover the exposed surface. Newly formed endothelial cells have also been known to cover the inner surface of plastic tubing used by surgeons to replace damaged blood vessels.

Endothelial cells not only repair damaged blood vessels, they also create new blood vessels. They do this in embryonic tissues to support growth, in normal adult tissue for repair and maintenance, and in damaged tissue to support repair. This process is called angiogenesis.

Angiogenesis is a critical component in embryonic development, tissue growth, tissue remodeling, and a number of pathologies. Angiogenesis results in the formation of new blood vessels. During angiogenesis, endothelial cells which exist in a quiescent state as part of an existing blood vessel grow and enter a migratory, proliferative state. This migratory, proliferative state is eventually resolved when the cells differentiate into capillary tubes and return to the quiescent state as part of a functional new blood vessel. Angiogenesis is orchestrated by a complex network of multiple macromolecular interactions.

Angiogenesis is regulated in both normal and malignant tissues by the balance of angiogenic stimuli and angiogenic inhibitors that are produced in the target tissue and at distant sites. Vascular endothelial growth factor-A (VEGF, also known as vascular permeability factor, VPF) is a primary stimulant of angiogenesis. VEGF is a multifunctional cytokine that is induced by hypoxia and oncogenic mutations and can be produced by a wide variety of tissues.

Angiogenesis is stimulated and harnessed by some neoplasms (e.g., tumors) to increase nutrient uptake. However, in contrast to normal angiogenesis, which leads to anastomoses (i.e., vessel connections) and capillary maturation, angiogenesis associated with neoplasia is typically a continuous process where vessel maturation is imperfect. Endothelial cells are activated by nearby neoplastic cells to secrete not only VEGF which stimulates angiogenesis, but also matrix metalloproteases (MMP) which degrade the surrounding extracellular matrix. The endothelial cells then invade the extracellular matrix where they proliferate, migrate, and organize to form new blood vessels, which support neoplasm growth and survival.

The newly vascularized neoplasm continues to grow, leading to further nutrient deprivation and chronic pro-angiogenic signaling. The vasculature of neoplasms is characterized by the presence of structural irregularities (lacunae) and a low rate of formation of inter-vessel connections. This incomplete vasculature is inefficient, such that often tumors require continuous angiogenesis to sustain themselves. Such imperfect vasculature is also believed to promote the shedding of neoplastic cells into the systemic circulation. Hence, the angiogenic potential of a neoplasm correlates with metastatic potential. As a significant proportion of neoplasms are dependent on continued angiogenesis, inhibition of angiogenesis blocks neoplasm growth which often leads to complete necrosis of the neoplasm.

Glial cells including astrocytes comprise a large proportion of the total cell population in the central nervous system. Unlike neurons, glial cells retain the ability to proliferate postnatally, and some glial cells still proliferate in the adult or aged brain. Uncontrolled glial proliferation can lead to aggressive primary intracranial tumors. Such tumors vary widely in morphology and behavior, and, according to the 1993 World Health Organization classification schema, can be separated into three subsets. Astrocytomas, the lowest grade tumors, are generally well-differentiated and tend to grow slowly. Anaplastic astrocytomas are characterized by increased cellularity, nuclear pleomorphism (ability to assume different forms), and increased mitotic activity. They are intermediate grade tumors and show a tendency to progress to a more aggressive grade. Glioblastoma cells are considered the most aggressive, with poorly differentiated cells, vascular proliferation, and necrosis. Glioblastoma U251 is a malignant cell line derived from the human glial cells.

The angiogenic effects of glioblastoma cells in the presence of in vivo matrix effects and other in vivo ancillary factors, do not predict in vitro effectiveness in inducing primary endothelial cells in culture to grow, and more particularly do not predict in vitro effectiveness in inducing primary endothelial cells in culture to form tubules. The effectiveness of very low numbers of tumor cells to induce this effect is still more unexpected.

Non-normal, i.e., immortalized endothelial cells have been reported to form tubular structures in culture in the presence of glioblastoma cells. But the growth of these kinds of cells can be expected to differ considerably from normal cells, so the induction of tubules in normal cells could not be reliably predicted from this earlier work. Lafleur, M.A. et al., J. Cell Science, 115(17):3427-3438 (2002) discloses the induction of tubulogenesis in human umbilical vein endothelial cells or human dermal microvascular endothelial cells co-cultured with U87 or U251 glioma cells for several days at 37°C und 5% CO₂ atmosphere apparently with a 1:1 ratio. Abe T. et al., J. Clin. Invest. 92(1):54-61 (1993) discloses the induction of tubulogenesis in bovine aortic endothelial cells co-cultured with glioma cells (including U251 cells) for three days. Nakagawa, M. et al., British Journal of Urology, 79(5):681-687 (1997) discloses the induction of tubulogenesis in human omental microvascular endothelial cells co-cultured with A498 renal cell carcinoma (RCC) cells for three days, and suggests the use of the co-cultured system for screening of inhibitors of tumor angiogenesis in RCC.

Thus, there is a need to induce angiogenic endothelial cells to better enable collection of angiogenic cells for such purposes as angiogenic assay kits and in the study of endothelial cells, particularly the functions and permeability of the endothelial cell barrier. Further, such cells have potential therapeutic uses

### Summary of the Invention

Described is a method of inducing tubulogenesis in normal endothelial cells. The method comprises co-culturing tumor cells with normal endothelial cells, wherein the ratio of endothelial cells to tumor cells is 5:1 or more. In a preferred embodiment the tumor cells comprise glioblastoma cells such as glioblastoma U251 or engineered or selected derivatives of glioblastoma U251 (e.g., transfected with VEGF). In a more preferred method the glial cells are glioblastoma cells, which preferably comprise glioblastoma U251 cells. The normal endothelial cells comprise, in a preferred embodiment, human endothelial cells that have not been immortalized. These include but are not limited to human dermal microvascular endothelial cells, human pulmonary microvascular endothelial cells or human umbilical vein endothelial cells.

In a preferred embodiment of the method the step of co-culturing the tumor cells and normal endothelial cells comprises incubating the endothelial cells and glial cells in an endothelial cell culture medium for at least 1 day and forming tubules from the incubated endothelial cells.

### Brief Description of the Drawings

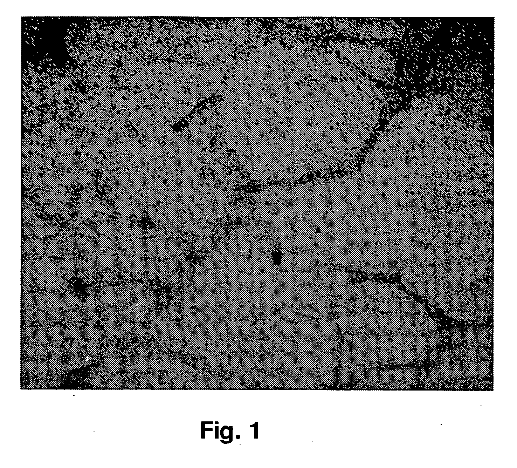

Figure 1 shows tubule formation by HMVEC cells in co-culture with glioblastoma cells.

### Detailed Description of the Invention

For simplicity and illustrative purposes, the principles of the present invention are described by referring to various exemplary embodiments thereof. The terminology used herein is for the purpose of description. Further, although certain methods are described with reference to certain steps that are presented herein in certain order, in many instances, these steps may be performed in any order as may be appreciated by one skilled in the art, and the methods are not limited to the particular arrangement of steps disclosed herein.

Endothelial cells are the cells that make up the inside of blood vessels. The term is used to incorporate human, other mammalian and other vertebrate endothelial cells. These include but are not limited to human dermal microvascular endothelial cells and human pulmonary microvascular endothelial cells (HMVEC) and human umbilical vein endothelial cells (HUVEC). Normal endothelial cells are endothelial cells that have not been immortalized. Mammalian cells, particularly human cells, are preferred. Selected normal endothelial cells comprise normal endothelial cells that are believed to have relatively high angiogenesis potential.

The method comprises co-culturing tumor cells with normal endothelial cells. In a preferred embodiment the method comprising co-culturing tumor cells with endothelial cells, forming tubules from the normal endothelial cells and selectively collecting the tubules of normal endothelial cells.

According to the invention the co-culturing is performed at an endothelial cell to U251 ratio of 5:1 or more, or preferably 50:1 or more.

In an embodiment of the present invention endothelial cells and glioblastoma U251 are harvested then suspended in an endothelial cell culture medium. The desired number of each cell type is added to a microwell and incubated. In a preferred embodiment the incubation occurs for more than 1 day. In a more preferred embodiment the incubation lasts for more than 2 days. In a preferred embodiment incubation occurs in a 5% CO₂ incubator at 37 degrees Celsius.

Tumor cells that are best candidates for use in the method are those that are known to induce angiogenesis.

The method of inducing tubulogenesis can be incorporated into a method of identifying bioactive agents by contacting prospective bioactive agents with the endothelial cells at some time relevant to modulating tubule formation. This time frame can be readily determined experimentally. Such time frame can be before the endothelial cells are co-cultured with tumor cells, or during the co-culturing. Bioactive agents that reduce tubulogenesis are candidate bioactive agents for disrupting blood vessel formation (angiogenesis) sought to be induced by tumor cells. Bioactive agents that stimulate tubulogenesis are candidate bioactive agents to increase vascularization in tissue damaged by ischemic events, in tissue whose vascularization has been damaged by environmental factors such as smoking, and in and aged-related blindness. Tubulogenesis can be most simply monitored visually, but can also be monitored by assaying the levels of molecules that function as surrogate markers for tubulogenesis.

A bioactive agent is a substance such as a chemical that can act on a cell, tissue, organ or organism, including but not limited to insecticides or drugs (i.e., pharmaceuticals) to create a change in the functioning of the cell, organ or organism.

A tubule formation altering effective amount of a bioactive agent is an amount effective to change the rate (to faster or slower), degree (greater or lesser) or morphology of tubule formation, or to change (upwards or downwards) another measure of tubulogenesis.

### Example

Two types of primary human endothelial cells, HUVEC and HMVEC were each are mixed at a ratio of 100:1 with tumor cell U251. The cells were seeded in 6-well plates at a density of 1×10⁶/well. The co-cultures were incubated in endothelial cell medium EGM-2 MV (Cambrex, Walkersville, MD) in a 37°C /5% CO₂ incubator and the medium was replaced every two days till day 11. Tube formation was observed on and after day 7 in both co-cultures. A HMVEC co-culture is shown in Figure 1.

## Claims

1. A method of inducing tubulogenesis in normal endothelial cells comprising co-culturing the normal endothelial cells with tumor cells and forming tubules from the normal endothelial cells, wherein the ratio of endothelial cells to tumor cells is 5:1 or more.

2. The method of claim 1, wherein the normal endothelial cells are human endothelial cells.

3. The method of claim 1, wherein the tumor cells comprise glioblastoma cells.

4. The method of claim 2, wherein the glioblastoma cells comprise glioblastoma U251.

5. The method of claim 4, wherein the step of co-culturing comprises:
incubating the endothelial cells and glioblastoma U251 in an endothelial cell culture medium for at least 1 day; and,
forming tubules from the incubated endothelial cells.

6. The method of claim 5, wherein the step of incubating the endothelial and tumor cells occurs in a 5% CO₂ incubator at 37° C.

7. The method of claim 2, wherein the human endothelial cells are human dermal microvascular endothelial cells, human pulmonary microvascular endothelial cells or human umbilical vein endothelial cells.

8. The method of claim 1, wherein the step of co-culturing comprises:
incubating the endothelial and tumor cells in an endothelial cell culture medium for at least 1 day; and,
forming tubules from the incubated endothelial cells.

9. The method of claim 8, wherein the step of incubating the endothelial and tumor cells is performed for at least 2 days.

10. The method of claim 8, wherein the step of incubating the endothelial and tumor cells occurs in a 5% CO₂ incubator at 37° C.

11. The method of claim 1 or 2, wherein the ratio of endothelial cells to tumor cells is 50:1 or more.

12. A method of collecting selected normal endothelial cells comprising:
a) co-culturing normal endothelial cells with tumor cells wherein the ratio of human endothelial cells to tumor cells is 5:1 or more;
b) forming tubules from the normal endothelial cells; and,
c) selectively collecting the tubules comprising normal endothelial cells.

13. A method of identifying bioactive agents comprising:
a) co-culturing normal endothelial cells with tumor cells and thereby forming tubules from the normal endothelial cells wherein the ratio of human endothelial cells to tumor cells is 5:1 or more;
b) contacting the normal endothelial cells with a prospective bioactive agent;
c) monitoring for changes in tubule formation resulting from the contacting.

14. The method of claim 12 or 13, wherein the normal endothelial cells are human endothelial cells.

15. The method of claim 12 or 13, wherein the tumor cells comprise glioblastoma cells.

16. The method of claim 15, wherein the glioblastoma cells comprise glioblastoma U251.

17. The method of claim 16, wherein the step of co-culturing comprises:
incubating the endothelial cells and glioblastoma U251 in an endothelial cell culture medium for at least 1 day; and,
forming tubules from the incubated endothelial cells.

18. The method of claim 14, wherein the human endothelial cells are human dermal microvascular endothelial cells, human pulmonary microvascular endothelial cells or human umbilical vein endothelial cells.

19. The method of claim 12 or 13, wherein the step of co-culturing comprises:
incubating the endothelial and tumor cells in an endothelial cell culture medium for at least 1 day; and,
forming tubules from the incubated endothelial cells.

20. The method of claim 19, wherein the step of incubating the endothelial and tumor cells is performed for at least 2 days.

## Patentansprüche

1. Verfahren zur Induzierung einer Tubulogenese in normalen Endothelialzellen, umfassend ein Co-Kultivieren der normalen Endothelialzellen mit Tumorzellen und die Bildung von Tubuli aus den normalen Endothelialzellen, wobei das Verhältnis von Endothelialzellen zu Tumorzellen 5:1 oder mehr beträgt.

2. Verfahren nach Anspruch 1, wobei die normalen Endothelialzellen humane Endothelialzellen sind.

3. Verfahren nach Anspruch 1, wobei die Tumorzellen Glioblastomzellen umfassen.

4. Verfahren nach Anspruch 2, wobei die Glioblastomzellen Glioblastom U251 umfassen.

5. Verfahren nach Anspruch 4, wobei der Schritt des Co-Kultivierens Folgendes umfasst:
Inkubieren der Endothelialzellen und von Glioblastom U251 in einem Endothelialzellen-Kulturmedium für wenigstens 1 Tag und
Bildung von Tubuli aus den inkubierten Endothelialzellen.

6. Verfahren nach Anspruch 5, wobei der Schritt des Inkubierens der Endothelial- und Tumorzellen in einem 5-%-CO₂-Inkubator bei 37 °C erfolgt.

7. Verfahren nach Anspruch 2, wobei die humanen Endothelialzellen humane dermale mikrovaskuläre Endothelialzellen, humane pulmonale mikrovaskuläre Endothelialzellen oder humane umbilikale Venen-Endothelialzellen sind.

8. Verfahren nach Anspruch 1, wobei der Schritt des Co-Kultivierens Folgendes umfasst:
Inkubieren der Endothelial- und der Tumorzellen in einem Endothelialzellen-Kulturmedium für wenigstens 1 Tag und
Bildung von Tubuli aus den inkubierten Endothelialzellen.

9. Verfahren nach Anspruch 8, wobei der Schritt des Inkubierens der Endothelial- und der Tumorzellen für wenigstens 2 Tage durchgeführt wird.

10. Verfahren nach Anspruch 8, wobei der Schritt des Inkubierens der Endothelial- und der Tumorzellen in einem 5-%-CO₂-Inkubator bei 37 °C erfolgt.

11. Verfahren nach Anspruch 1 oder 2, wobei das Verhältnis von Endothelialzellen zu Tumorzellen 50: 1 oder mehr beträgt.

12. Verfahren zur Isolierung von ausgewählten normalen Endothelialzellen, umfassend:
a) Co-Kultivieren von normalen Endothelialzellen mit Tumorzellen, wobei das Verhältnis von humanen Endothelialzellen zu Tumorzellen 5:1 oder mehr beträgt,
b) Bildung von Tubuli aus den normalen Endothelialzellen und
c) selektive Isolierung der normale Endothelialzellen umfassenden Tubuli.

13. Verfahren zur Identifizierung von bioaktiven Mitteln, umfassend:
a) Co-Kultivieren von normalen Endothelialzellen mit Tumorzellen und **dadurch** die Bildung von Tubuli aus den normalen Endothelialzellen, wobei das Verhältnis von humanen Endothelialzellen zu Tumorzellen 5: 1 oder mehr beträgt,
b) In-Kontakt-Bringen der normalen Endothelialzellen mit einem potenziellen bioaktiven Mittel,
c) Überprüfen auf Änderungen einer aus dem In-Kontakt-Bringen resultierenden Tubulusbildung.

14. Verfahren nach Anspruch 12 oder 13, wobei die normalen Endothelialzellen humane Endothelialzellen sind.

15. Verfahren nach Anspruch 12 oder 13, wobei die Tumorzellen Glioblastomzellen umfassen.

16. Verfahren nach Anspruch 15, wobei die Glioblastomzellen Glioblastom U251 umfassen.

17. Verfahren nach Anspruch 16, wobei der Schritt des Co-Kultivierens Folgendes umfasst:
Inkubieren der Endothelialzellen und des Glioblastoms U251 in einem Endothelialzellen-Kulturmedium für wenigstens 1 Tag und
Bildung von Tubuli aus den inkubierten Endothelialzellen.

18. Verfahren nach Anspruch 14, wobei die humanen Endothelialzellen humane dermale mikrovaskuläre Endothelialzellen, humane pulmonale mikrovaskuläre Endothelialzellen oder humane umbilikale Venen-Endothelialzellen sind.

19. Verfahren nach Anspruch 12 oder 13, wobei der Schritt des Co-Kultivierens Folgendes umfasst:
Inkubieren der Endothelialzellen und der Tumorzellen in einem Endothelialzellen-Kulturmedium für wenigstens 1 Tag und
Bildung von Tubuli aus den inkubierten Endothelialzellen.

20. Verfahren nach Anspruch 19, wobei der Schritt des Inkubierens der Endothelial- und der Tumorzellen für wenigstens 2 Tage durchgeführt wird.

## Revendications

1. Procédé d'induction de la tubulogenèse dans des cellules endothéliales normales, comprenant les étapes consistant à cocultiver les cellules endothéliales normales avec des cellules tumorales et à former des tubules avec les cellules endothéliales normales, dans lequel le rapport des cellules endothéliales aux cellules tumorales est de 5/1 ou plus.

2. Procédé selon la revendication 1, dans lequel les cellules endothéliales normales sont des cellules endothéliales humaines.

3. Procédé selon la revendication 1, dans lequel les cellules tumorales comprennent les cellules de glioblastome.

4. Procédé selon la revendication 3, dans lequel les cellules de glioblastome comprennent les U251 de glioblastome.

5. Procédé selon la revendication 4, dans lequel l'étape de coculture comprend les étapes consistant à :
incuber les cellules endothéliales et les U251 de glioblastome dans un milieu de culture de cellules endothéliales pendant au moins 1 jour ; et,
former des tubules avec les cellules endothéliales incubées.

6. Procédé selon la revendication 5, dans lequel l'étape qui consiste à incuber les cellules endothéliales et tumorales a lieu dans un incubateur contenant 5 % de CO₂, à 37°C.

7. Procédé selon la revendication 2, dans lequel les cellules endothéliales humaines sont des cellules endothéliales microvasculaires dermiques humaines, des cellules endothéliales microvasculaires pulmonaires humaines ou des cellules endothéliales de veine ombilicale humaines.

8. Procédé selon la revendication 1, dans lequel l'étape de coculture comprend les étapes consistant à :
incuber les cellules endothéliales et tumorales dans un milieu de culture de cellules endothéliales pendant au moins 1 jour ; et,
former des tubules avec les cellules endothéliales incubées.

9. Procédé selon la revendication 8, dans lequel l'étape consistant à incuber les cellules endothéliales et tumorales est réalisée pendant au moins 2 jours.

10. Procédé selon la revendication 8, dans lequel l'étape consistant à incuber les cellules endothéliales et tumorales a lieu dans un incubateur contenant 5 % de CO₂, à 37°C.

11. Procédé selon la revendication 1 ou 2, dans lequel le rapport des cellules endothéliales aux cellules tumorales est de 50/1 ou plus.

12. Procédé de collecte de cellules endothéliales normales choisies, comprenant les étapes qui consistent à :
a) cocultiver des cellules endothéliales normales avec des cellules tumorales, le rapport des cellules endothéliales humaines aux cellules tumorales étant de 5/1 ou plus ;
b) former des tubules avec les cellules endothéliales normales ; et,
c) collecter de façon sélective les tubules comprenant les cellules endothéliales normales.

13. Procédé d'identification d'agents bioactifs comprenant les étapes qui consistent à :
a) cocultiver des cellules endothéliales normales avec des cellules tumorales et former de cette façon des tubules avec les cellules endothéliales normales, le rapport des cellules endothéliales humaines aux cellules tumorales étant de 5/1 ou plus ;
b) mettre les cellules endothéliales normales en contact avec un agent bioactif potentiel ;
c) pratiquer une surveillance afin de déceler les changements, au niveau de la formation de tubules, qui résultent de la mise en contact.

14. Procédé selon la revendication 12 ou 13, dans lequel les cellules endothéliales normales sont des cellules endothéliales humaines.

15. Procédé selon la revendication 12 ou 13, dans lequel les cellules tumorales comprennent les cellules de glioblastome.

16. Procédé selon la revendication 15, dans lequel les cellules de glioblastome comprennent les U251 de glioblastome.

17. Procédé selon la revendication 16, dans lequel l'étape de coculture comprend les étapes consistant à :
incuber les cellules endothéliales et les U251 de glioblastome dans un milieu de culture de cellules endothéliales pendant au moins 1 jour ; et,
former des tubules avec les cellules endothéliales incubées.

18. Procédé selon la revendication 14, dans lequel les cellules endothéliales humaines sont des cellules endothéliales microvasculaires dermiques humaines, des cellules endothéliales microvasculaires pulmonaires humaines ou des cellules endothéliales de veine ombilicale humaines.

19. Procédé selon la revendication 12 ou 13, dans lequel l'étape de coculture comprend les étapes consistant à :
incuber les cellules endothéliales et tumorales dans un milieu de culture de cellules endothéliales pendant au moins 1 jour ; et,
former des tubules avec les cellules endothéliales incubées.

20. Procédé selon la revendication 19, dans lequel l'étape consistant à incuber les cellules endothéliales et tumorales est réalisée pendant au moins 2 jours.
